# EUROPEAN PATENT APPLICATION

(11) **EP 3 970 651 A1**
(43) Date of publication of application: **23.03.2022**
(21) Application number: 21197105.6
(22) Date of filing: 16.09.2021
(51) Int. Cl.: A61B 34/30, A61B 34/37, A61B 18/14, A61B 18/00, A61B 90/00

(54) **MULTI-FUNCTION SURGICAL INSTRUMENTS**

(30) Priority: 16.09.2020 US 202017022205
(71) Applicant: Covidien LP, Mansfield, MA 02048 (US)
(72) Inventor: BAGROSKY, Tyler, J, Boulder, CO Colorado 80301 (US); MCHENRY, Jennifer, R, Boulder, CO Colorado 80301 (US); JOSEPH, Daniel, A, Boulder, CO Colorado 80301 (US); BOUCHER, Todd, W, Boulder, CO Colorado 80301 (US); SANDERS, Jason, T, Boulder, CO Colorado 80301 (US)
(74) Representative: Maschio, Antonio

(57) **Abstract**

A surgical instrument includes an elongated shaft supporting an end effector assembly, a sheath slidably disposed about the elongated shaft and the end effector assembly, and a housing disposed at a proximal end portion of the elongated shaft. Inflow, outflow, and common tubes extend into the housing. A manifold is disposed within the housing and defines an inflow port fluidly coupled to the inflow tube, and outflow port fluidly coupled to the outflow tube, and a common tube port fluidly coupled to the common tube. First and second valve assemblies are disposed within the housing and operably coupled to the manifold. The first valve assembly is configured to selectively control a flow of fluid from the inflow tube to the interior of the sheath, and the second valve assembly is configured to selectively control a flow of fluid from the interior of the sheath to the outflow tube.

## Description

### CROSS REFERENCE TO RELATED APPLICATIONS

This application is a continuation-in-part of U.S. Patent Application No. 16/899,661, filed on June 12, 2020, which claims the benefit of U.S. Provisional Application Serial No. 62/867,292, filed June 27, 2019, the entire contents of each of which are hereby incorporated by reference herein.

### FIELD

The present disclosure relates to surgical instruments and, more specifically, to multi-function surgical instruments.

### BACKGROUND

In minimally-invasive surgical procedures, operations are carried out within an internal body cavity through small entrance openings in the body. The entrance openings may be natural passageways of the body or may be surgically created, for example, by making a small incision into which a cannula is inserted.

Multi-function surgical instruments are beneficial in that they allow multiple surgical tasks to be performed with a single instrument, obviating the need to alternatingly remove and insert different instruments into the surgical site to perform a surgical task and/or obviating the need for simultaneously inserting multiple instruments into the surgical site to perform a surgical task.

### SUMMARY

As used herein, the term "distal" refers to the portion that is described which is further from an operator (whether a human surgeon or a surgical robot), while the term "proximal" refers to the portion that is being described which is closer to the operator. Further, any or all of the aspects described herein, to the extent consistent, may be used in conjunction with any or all of the other aspects described herein.

Provided in accordance with aspects of the present disclosure is a surgical instrument including an end effector assembly including first and second jaw members. At least one of the first or second jaw members is movable relative to the other between a spaced-apart position and an approximated position for grasping tissue therebetween. A sheath is movable relative to the end effector assembly between a retracted position, wherein the sheath is positioned proximally of the first and second jaw members, and an extended position, wherein the sheath is disposed about the first and second jaw members. The sheath is configured to fluidly couple to a source of at least one of suction or irrigation to provide at least one of suction or irrigation at a surgical site.

In an aspect of the present disclosure, the surgical instrument further includes a housing and a shaft extending distally from the housing. In such aspects, the end effector assembly is supported at a distal end of the shaft and the sheath is slidably disposed about the shaft.

In another aspect of the present disclosure, a fluid port is disposed on the housing and configured to connect to the source of suction and/or irrigation. In such aspects, an internal fluid line disposed within the housing fluidly couples the sheath with the fluid port.

In still another aspect of the present disclosure, at least one actuator disposed on the housing is selectively actuatable for deploying and retracting the sheath.

In yet another aspect of the present disclosure, a movable handle extends from the housing and couples to the at least one of the first or second jaw members. The movable handle is selectively actuatable to move the at least one of the first or second jaw members relative to the other.

In still yet another aspect of the present disclosure, the sheath defines an open distal end and is configured to provide the at least one of suction or irrigation through the open distal end thereof. Alternatively or additionally, the sheath defines a plurality of apertures through a side wall thereof and is configured to provide the at least one of suction or irrigation through the plurality of apertures.

In another aspect of the present disclosure, the surgical instrument further includes an energizable member coupled to the sheath and extending distally therefrom. The energizable member is configured to move with the sheath between the retracted position, wherein the energizable member is positioned proximally of the first and second jaw members, and an extended position, wherein the energizable member extends distally from the first and second jaw members.

In yet another aspect of the present disclosure, the energizable member is configured to selectively supply monopolar energy to tissue.

In still another aspect of the present disclosure, the energizable member defines a hook-shaped configuration or a spatula-shaped configuration.

In another aspect of the present disclosure, the surgical instrument further includes a fluid jet member coupled to the sheath and extending distally therefrom. The fluid jet member is configured to move with the sheath between the retracted position, wherein the fluid jet member is positioned proximally of the first and second jaw members, and an extended position, wherein the fluid jet member extends distally from the first and second jaw members. The fluid jet member is configured to provide a fluid jet stream.

In still yet another aspect of the present disclosure, at least one of the first or second jaw members is configured to connect to a source of energy for treating tissue grasped therebetween. In such aspects, the first and second jaw members may be configured to connect to a source of bipolar electrosurgical energy for conducting energy therebetween to treat tissue grasped therebetween.

Also provided in accordance with the present disclosure is a surgical instrument including an elongated shaft, an end effector assembly supported at a distal end portion of the elongated shaft, a sheath disposed about the elongated shaft and movable relative to the elongated shaft and the end effector assembly between a retracted position and an extended position, a housing disposed at a proximal end portion of the elongated shaft, an inflow tube extending into the housing, an outflow tube extending into the housing, a common tube extending into the housing, a manifold disposed within the housing, and first and second valve assemblies. The common tube is fluidly coupled to an interior of the sheath and configured to supply fluid thereto and remove fluid therefrom. The manifold defines an inflow port fluidly coupled to the inflow tube, an outflow port fluidly coupled to the outflow tube, and a common tube port fluidly coupled to the common tube. The first and second valve assemblies are disposed within the housing and operably coupled to the manifold. The first valve assembly is configured to selectively control a flow of fluid from the inflow tube to the interior of the sheath. The second valve assembly is configured to selectively control a flow of fluid from the interior of the sheath to the outflow tube.

In an aspect of the present disclosure, each of the first and second valve assemblies includes a solenoid valve. In such aspects, a selector assembly including first and second switches configured to selectively operate the solenoid valves of the first and second valve assemblies, respectively, may be provided. Further, a toggle switch may be disposed on the housing and configured to selectively actuate the first and seconds switches.

In another aspect of the present disclosure, the elongated shaft defines a C-shaped configuration and includes a side-facing opening defined therein along a portion of a length thereof. In such aspects, at least one drive component associated with the end effector assembly may extend through the elongated shaft.

In yet another aspect of the present disclosure, the elongated shaft defines a flared distal portion.

In still another aspect of the present disclosure, the sheath includes an expandable distal portion. In such aspects, an expandable structure may be disposed within the expandable distal portion of the sheath and configured to selectively expand the expandable distal portion of the sheath.

In still yet another aspect of the present disclosure, at least one flow director is disposed within the sheath adjacent the end effector assembly. The at least one flow director is configured to direct a flow of fluid through the sheath around the end effector assembly.

A robotic surgical system provided in accordance with the present disclosure includes a robotic arm including a plurality of tube guides disposed thereon, and a robotic surgical instrument configured to releasably engage the robotic arm. The robotic surgical instrument includes a housing, an elongated shaft extending distally from the housing, an end effector assembly supported at a distal end portion of the elongated shaft, a sheath disposed about the elongated shaft and movable relative to the elongated shaft and the end effector assembly between a retracted position and an extended position, an inflow tube routed through at least one of the plurality of tube guides and extending into the housing, an outflow tube routed through at least one of the plurality of tube guides and extending into the housing, and first and second valve assemblies disposed within the housing and operably coupled to the inflow and outflow tubes, respectively. The first valve assembly is configured to selectively control a flow of fluid from the inflow tube to the interior of the sheath, and the second valve assembly is configured to selectively control a flow of fluid from the interior of the sheath to the outflow tube.

In an aspect of the present disclosure, the robotic surgical instrument further includes a common tube extending into the housing and fluidly coupled to an interior of the sheath to supply fluid thereto and remove fluid therefrom, and a manifold disposed within the housing and defining an inflow port fluidly coupled to the inflow tube, and outflow port fluidly coupled to the outflow tube, and a common tube port fluidly coupled to the common tube.

In another aspect of the present disclosure, each of the first and second valve assemblies includes a solenoid valve. In such aspects, first and second switches disposed within the housing and configured to selectively operate the solenoid valves of the first and second valve assemblies, respectively, may be provided.

In still another aspect of the present disclosure, the housing includes a main housing body and a fluid control box. In such aspects, the first and second valve assemblies may be disposed within the fluid control box.

In yet another aspect of the present disclosure, the elongated shaft defines a C-shaped configuration and includes a side-facing opening defined therein along a portion of a length thereof. Additionally or alternatively, the elongated shaft defines a flared distal portion.

In still yet another aspect of the present disclosure, at least one drive component associated with the end effector assembly extends through the elongated shaft.

In an aspect of the present disclosure, the sheath includes an expandable distal portion and an expandable structure disposed within the expandable distal portion to selectively expand the expandable distal portion of the sheath.

In another aspect of the present disclosure, at least one flow director is disposed within the sheath adjacent the end effector assembly to direct a flow of fluid through the sheath around the end effector assembly.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements.
FIG. 1 is a perspective view of a multi-function surgical instrument in accordance with aspects the present disclosure;
FIG. 2 is an enlarged, perspective view of a distal end portion of the surgical instrument of FIG. 1 wherein a deployable assembly thereof is disposed in a retracted position;
FIG. 3 is an enlarged, perspective view of the distal end portion of the surgical instrument of FIG. 1, wherein the deployable assembly thereof is disposed in a deployed position;
FIG. 4 is a perspective view of a proximal end portion of the surgical instrument of FIG. 1 with portions removed to illustrate the internal working components thereof;
FIG. 5 is an enlarged, perspective view of the distal end portion of another surgical instrument provided in accordance with aspects of the present disclosure similar to the surgical instrument of FIG. 1, wherein the deployable assembly thereof is disposed in a deployed position;
FIG. 6 is an enlarged, perspective view of the distal end portion of yet another surgical instrument provided in accordance with aspects of the present disclosure similar to the surgical instrument of FIG. 1, wherein the deployable assembly thereof is disposed in a deployed position;
FIG. 7 is an enlarged, perspective view of the distal end portion of still another surgical instrument provided in accordance with aspects of the present disclosure similar to the surgical instrument of FIG. 1, wherein the deployable assembly thereof is disposed in a deployed position;
FIG. 8 is an enlarged, perspective view of the distal end portion of still yet another surgical instrument provided in accordance with aspects of the present disclosure similar to the surgical instrument of FIG. 1, wherein the deployable assembly thereof is disposed in a deployed position;
FIG. 9 is a perspective view of a proximal end portion of the surgical instrument of FIG. 8 with portions removed to illustrate the internal working components thereof;
FIG. 10 is a perspective view of another multi-function surgical instrument in accordance with aspects the present disclosure;
FIG. 11 is a side view, with a housing portion removed, of the multi-function surgical instrument of FIG. 10 shown incorporated into a surgical system in accordance with the present disclosure;
FIG. 12A is an enlarged, perspective view, with a housing portion removed, of the multi-function surgical instrument of FIG. 10;
FIG. 12B is an enlarged, side view of the area of detail indicated as "12B" in FIG. 11;
FIG. 13 is a transverse, cross-sectional view taken across section line "13-13" of FIG. 11;
FIG. 14 is an exploded, perspective view of the multi-function surgical instrument of FIG. 10;
FIG. 15 is an exploded, perspective view of a distal portion of the multi-function surgical instrument of FIG. 10;
FIG. 16 is a transverse, cross-sectional view taken across section line "16-16" of FIG. 15;
FIG. 17 is a longitudinal, cross-sectional view taken across section line "17-17" of FIG. 12;
FIG. 18 is an enlarged, longitudinal, cross-sectional view of the area of detail indicated as "18" in FIG. 17;
FIG. 19 is an enlarged, side view, with portions shown in phantom, of the end effector assembly of the multi-function surgical instrument of FIG. 10;
FIG. 20 is a schematic illustration of a robotic surgical system provided in accordance with aspects of the present disclosure;
FIG. 21 is a schematic illustration of a multi-function surgical instrument provided in accordance with the present disclosure and configured for use with the robotic surgical system of FIG. 20;
FIG. 22 is an enlarged, perspective view of a distal end portion configuration of a multi-function surgical instrument provided in accordance with the present disclosure; and
FIG. 23 is an enlarged, perspective view of another distal end portion configuration of a multi-function surgical instrument provided in accordance with the present disclosure.

### DETAILED DESCRIPTION

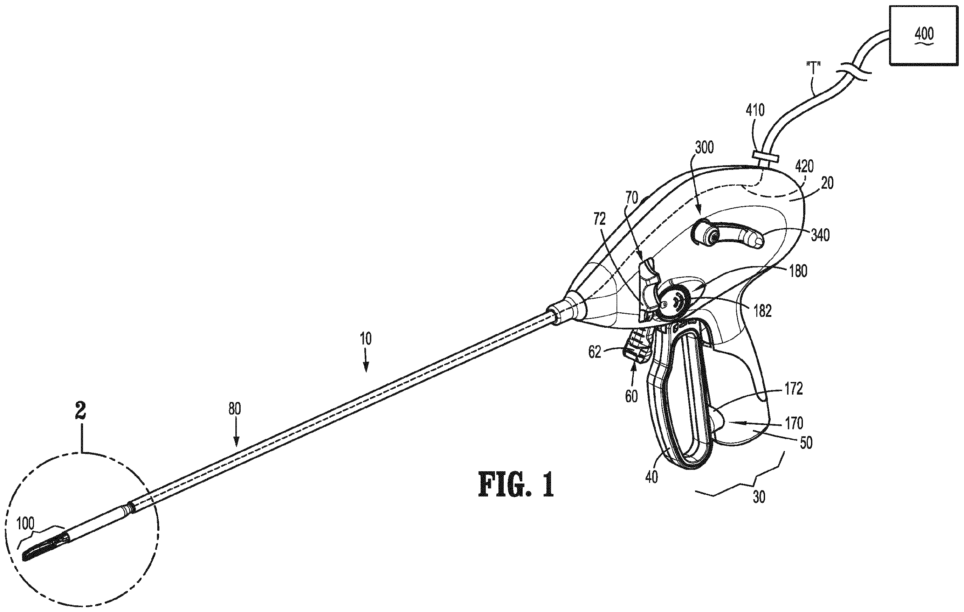

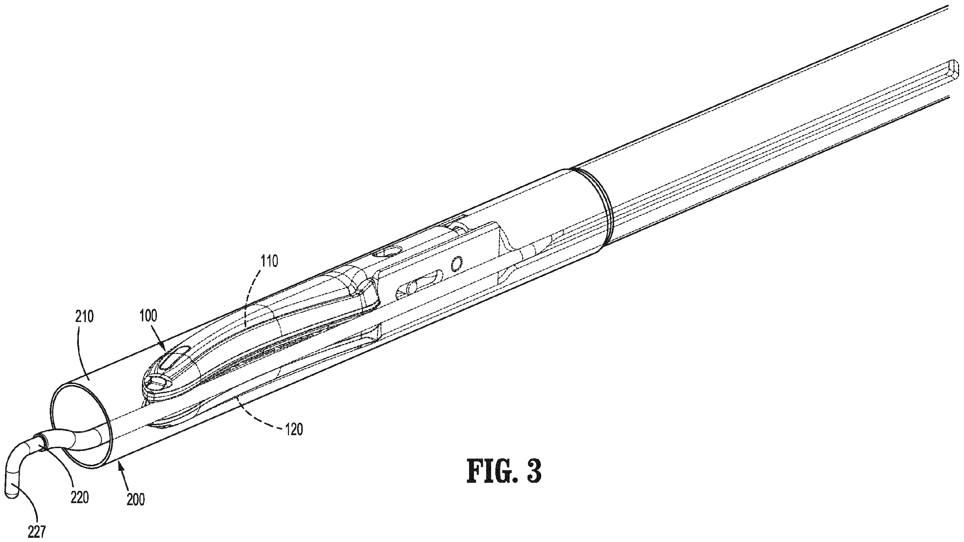

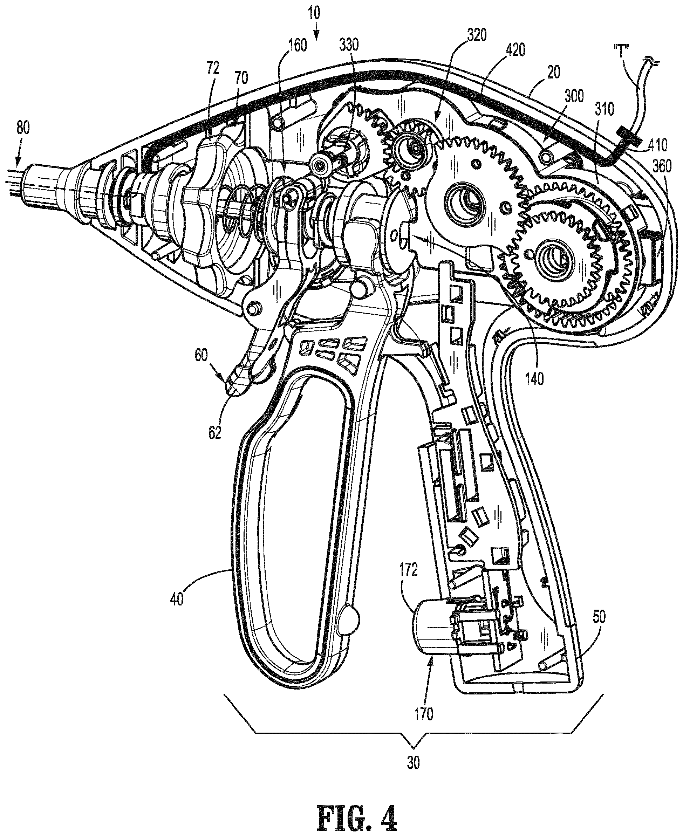

Referring generally to FIGS. 1-9, multi-function surgical instruments provided in accordance with the present disclosure are configured to operate in a first mode, e.g., for grasping tissue, treating (e.g., sealing) grasped tissue with bipolar energy, and/or mechanically dissecting grasped tissue, and a second mode, e.g., for treating tissue and/or electrically/ electromechanically dissecting tissue with monopolar, thermal, microwave, or other suitable energy. Instrument 10 further provides suction and/or irrigation capability before, during, after, and/or in place of the first and second modes of operation.

With reference to FIGS. 1-4, a multi-function surgical instrument provided in accordance with the present disclosure is shown generally identified by reference numeral 10. Instrument 10 includes a housing 20, a handle assembly 30, a trigger assembly 60, a rotation assembly 70, an elongated shaft assembly 80, an end effector assembly 100, a drive assembly 140, a knife assembly 160, first and second activation assemblies 170, 180, respectively, a deployable assembly 200, and a deployment and retraction mechanism 300.

Instrument 10 also includes an electrosurgical cable (not shown) that connects instrument 10 to a generator (not shown) or other suitable power source. The electrosurgical cable includes wires (not shown) extending therethrough that have sufficient length to extend through housing 20 and/or elongated shaft assembly 80 in order to provide energy to at least one of the electrically-conductive surfaces 112, 122 of jaw members 110, 120, respectively, of end effector assembly 100, e.g., upon activation of first activation switch 172 of first activation assembly 170 in the first mode of operation. Similarly, one or more of the wires of the electrosurgical cable extends through housing 20 and/or elongated shaft assembly 80 in order to provide energy to energizable member 220 of deployable assembly 200, e.g., upon activation of either of the second activation switches 182 of second activation assembly 180 in the second mode of operation.

Instrument 10 further includes a fluid port 410 disposed on housing 20 that enables connection of instrument 10 to a suction and/or irrigation source 400 via suitable tubing "T" (integral or removable tubing "T"). Within housing 20, an internal fluid line 420 connects fluid port 410 with the interior of sheath 210 of deployable assembly 200 to enable the delivery of fluid to and/or withdrawal of fluid from the interior of sheath 210. More specifically, suction and/or irrigation source 400 may be configured to only provide suction or irrigation through sheath 210. Alternatively, suction and/or irrigation source 400 may be configured to provide, in a first configuration, suction though sheath 210, and, in a second configuration, irrigation though sheath 210. Instrument 10 may further include controls (not shown) such as ON/OFF buttons, adjustable buttons, etc. for controlling suction and/or irrigation. The controls may be disposed on housing 20, on another portion of instrument 10, or may be remote from instrument 10, e.g., on the suction and/or irrigation source 400.

Elongated shaft assembly 80 extends distally from housing 20 and supports end effector assembly 100 at a distal end thereof. End effector assembly 100 includes opposing jaw members 110, 120 pivotably coupled to one another. Each of the jaw members 110, 120 includes an electrically-conductive surface 112, 122 adapted to connect to the source of energy and defines a bipolar configuration in use wherein surface 112 is charged to a first electrical potential and surface 122 is charged to a second, different electrical potential such that an electrical potential gradient is created for conducting energy between surfaces 112, 122 and through tissue grasped therebetween for treating tissue. First activation switch 172 of first activation assembly 170 (FIG. 1) is operably coupled between the source of energy (not shown) and surfaces 112, 122 via one or more wires (not shown), thus allowing the surgeon to apply energy, e.g., bipolar electrosurgical energy, to surfaces 112, 122 of jaw members 110, 120, respectively, of end effector assembly 100 during the fist mode of operation.

Handle assembly 30 includes a movable handle 40 and a fixed handle 50. Movable handle 40 is movable relative to fixed handle 50 between an initial position, wherein movable handle 40 is spaced-apart from fixed handle 50, and a compressed position, wherein movable handle 40 is compressed towards fixed handle 50. Drive assembly 140 is operably coupled between handle assembly 30 and end effector assembly 100 such that movement of movable handle 40 between the initial position and the compressed position pivots jaw member 110 relative to jaw member 120 between the spaced-apart position and the approximated position. Bilateral configurations of jaw members 110, 120 are also contemplated.

Continuing with reference to FIGS. 1-4, trigger 62 of trigger assembly 60 is selectively actuatable relative to housing 20 from an un-actuated position to an actuated position. Knife assembly 160 is operably coupled to trigger 62 such that actuation of trigger 62 from the un-actuated position to the actuated position translates a knife (not shown) of knife assembly 160 from a retracted position, wherein the knife (not shown) is disposed proximally of jaw members 110, 120, to an extended position, wherein the knife (not shown) extends at least partially between jaw members 110, 120 and through knife channels (not shown) defined within jaw members 110, 120 to cut tissue grasped between jaw members 110, 120.

Rotation of rotation wheel 72 of rotation assembly 70 relative to housing 20 effects corresponding rotation of elongated shaft assembly 80, end effector assembly 100, drive assembly 140, knife assembly 160, and deployable assembly 200 relative to housing 20.

Deployable assembly 200 includes a sheath 210 and an energizable member 220. Sheath 210, in configurations, is insulative, although other configurations are also contemplated. Sheath 210 is movable relative to end effector assembly 100 between a retracted position, wherein sheath 210 is disposed proximally of end effector assembly 100, and an extended position, wherein sheath 210 is substantially disposed about end effector assembly 100. Energizable member 220 is coupled to the source of energy (not shown) and second activation assembly 180 (FIG. 1) via one or more wires (not shown) and may function as the active electrode of a monopolar circuit or may be energizable with any other suitable form of energy, e.g., thermal, microwave, etc. Energizable member 220 is movable together with sheath 210 and relative to end effector assembly 100 between a retracted position, wherein distal tissue-treating portion 227 of energizable member 220 is positioned more-proximally, and an extended position, wherein distal tissue-treating portion 227 of energizable member 220 extends distally from end effector assembly 100 to facilitate treating tissue therewith. Energizable member 220, more specifically, is engaged with sheath 210 such that energizable member 220 and sheath 210 move together between their respective retracted and extended positions (collectively the retracted and extended positions of deployable assembly 200). In the extended position, in configurations where sheath 210 is insulative, sheath 210 serves to electrically insulate end effector assembly 100 from distal tissue-treating portion 227 of energizable member 220, while distal tissue-treating portion 227 extends distally from end effector assembly 100. In the extended position, energy may be supplied to distal tissue-treating portion 227 of energizable member 220, e.g., via activation of either of the activation switches 182 of second activation assembly 180 (FIG. 1), for treating tissue in the second mode of operation.

Sheath 210, as noted above, is coupled to suction and/or irrigation source 400 via internal fluid line 420, fluid port 410, and tubing "T" to enable the delivery of fluid to and/or withdrawal of fluid from the interior of sheath 210. As such, fluid may be suctioned from a surgical site into the open distal end of sheath 210 and through sheath 210 and/or may be supplied to the surgical site via the open distal end of sheath 210. Suction and/or irrigation may be provided in the retracted position of deployable assembly 200, in the extended position of deployable assembly 200, or in both the retracted and extended positions of deployable assembly 200. Thus, suction and/or irrigation may be provided before, during, and/or after use of instrument 10 in the first mode of operation and/or second mode of operation.

Deployment and retraction mechanism 300 is configured for selectively transitioning deployable assembly 200 between its retracted position and its extended position. Deployment and retraction mechanism 300 generally includes a gear box 310 mounted within housing 20, a gear assembly 320 operably disposed within gear box 310, a pair of input shafts 330 operably coupled to gear assembly 320 and extending transversely from either side of gear box 310 and outwardly from housing 20 through apertures defined through housing 20 (only one side of housing 20 and, thus, one input shaft 330 is illustrated), a pair of deployment paddles 340 operably coupled to the input shafts 330 (only one side of housing 20 and, thus, one paddle 340 is illustrated), and a slider 360 disposed within housing 20 and operably coupling an output of gear assembly 330 with energizable member 220 of deployable assembly 200 (which, in turn, is engaged with sheath 210) such that deployment and retraction mechanism 300 is configured to enable both deployment and retraction of deployable assembly 200 in a push-push manner, e.g., wherein deployable assembly 200 is both deployed and retracted by pushing either of paddles 340 in the same direction. Other suitable deployment mechanisms are also contemplated.

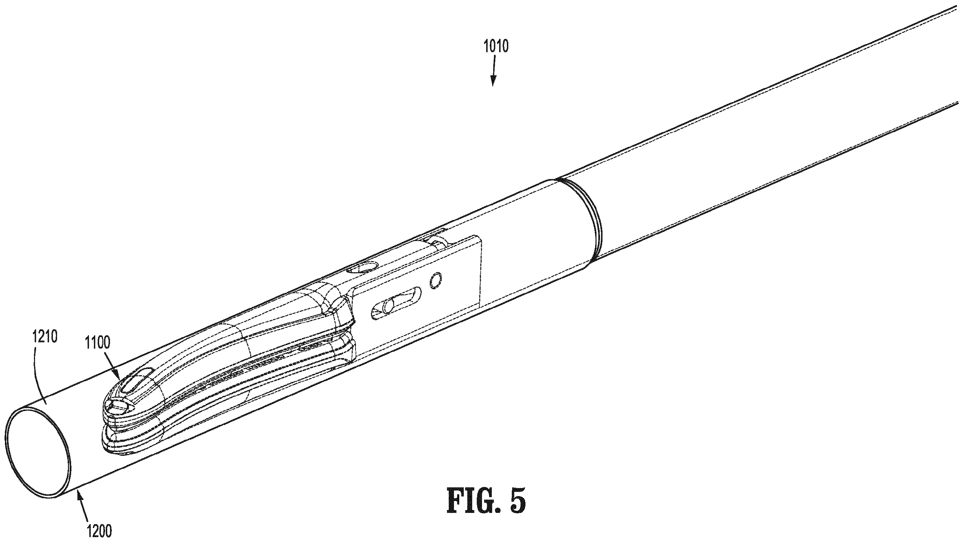

Referring to FIG. 5, another multi-function surgical instrument provided in accordance with the present disclosure is shown generally identified by reference numeral 1010. Instrument 1010 is similar to instrument 10 (FIG. 1) and may include any of the features thereof. Thus, for purposes of brevity, only the differences between instrument 1010 and instrument 10 (FIG. 1) are described in detail below while similarities are summarily described or omitted entirely.

Instrument 1010 is configured to operate in a first mode, e.g., for grasping tissue, treating grasped tissue with energy, and/or mechanically dissecting grasped tissue (similarly as detailed above with respect to instrument 10 (FIG. 1)), and a suction/irrigation mode, e.g., to provide suction and/or irrigation at a surgical site. More specifically, deployable assembly 1200 of instrument 1010 includes a sheath 1210 (but does not include an energizable member as with instrument 10 (FIG. 1)). Sheath 1210 is movable relative to end effector assembly 1100 between a retracted position, wherein sheath 1210 is disposed proximally of end effector assembly 1100, and an extended position, wherein sheath 1210 is substantially disposed about end effector assembly 1100.

Sheath 1210 of deployable assembly 1200 is coupled to a suction and/or irrigation source (see source 400 (FIG. 1)) to enable the delivery of fluid to and/or withdrawal of fluid from the surgical site via open distal end of sheath 1210. Suction and/or irrigation may be applied through sheath 1210 in the retracted position of sheath 1210, in the extended position of sheath 1200, or in both the retracted and extended positions of sheath 1210. Thus, suction and/or irrigation may be provided before, during, and/or after use of instrument 10 in the first mode of operation.

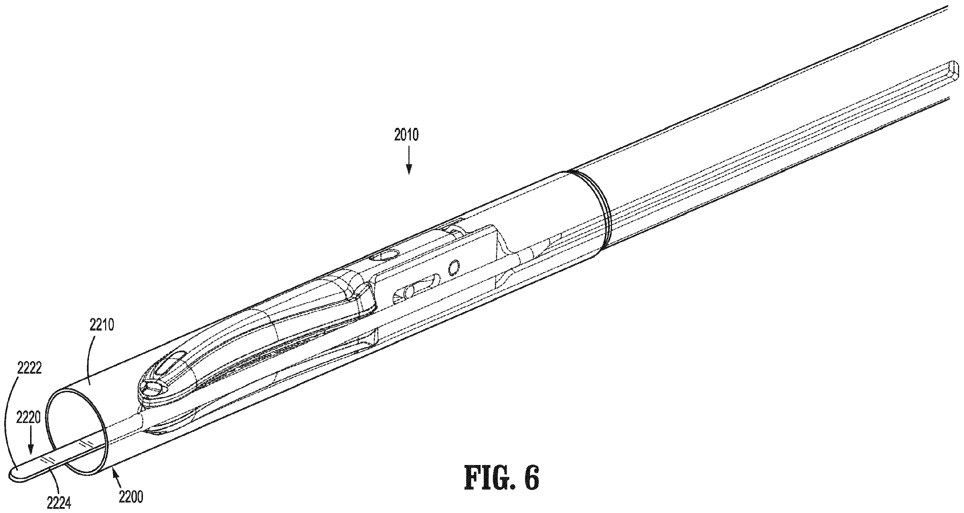

Turning to FIG. 6, another multi-function surgical instrument provided in accordance with the present disclosure is shown generally identified by reference numeral 2010. Instrument 2010 is similar to instrument 10 (FIG. 1) and may include any of the features thereof. Thus, for purposes of brevity, only the differences between instrument 2010 and instrument 10 (FIG. 1) are described in detail below while similarities are summarily described or omitted entirely.

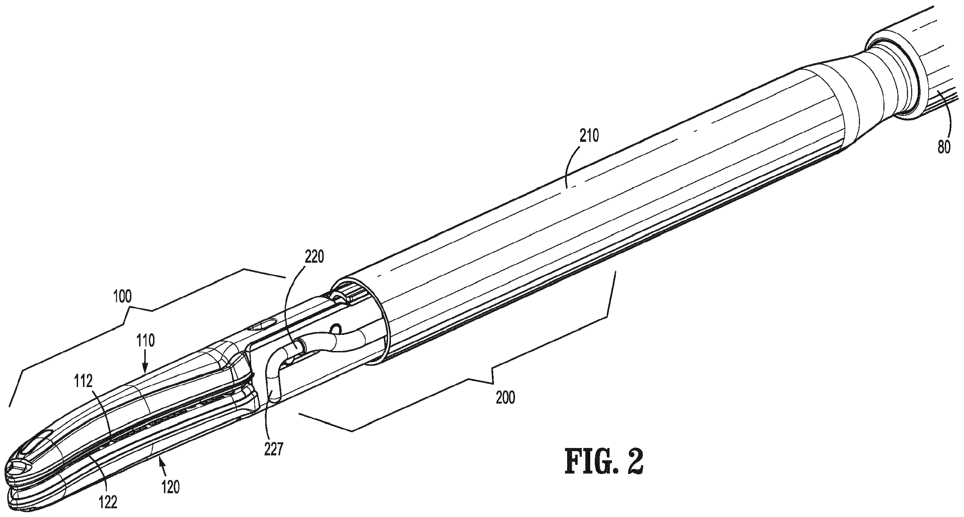

Deployable assembly 2200 of instrument 2010 includes a sheath 2210 and an energizable member 2220. Sheath 2210 of deployable assembly 2200 is coupled to a suction and/or irrigation source (see source 400 (FIG. 1)) to enable the delivery of fluid to and/or withdrawal of fluid from the interior of sheath 2210. Energizable member 2220 is coupled to the source of energy (not shown) and may function as the active electrode of a monopolar circuit or may be energizable with any other suitable form of energy, e.g., thermal, microwave, etc. Alternatively, energizable member 2220 is not energizable (and, thus, not coupled to a source of energy) but, rather, is configured as a mechanical component configured to facilitate manual, mechanical manipulation of tissue. Energizable member 2220 defines a spatula-shaped configuration including relatively broad opposing surfaces 2222 and relatively narrow peripheral edges 2224. However, other suitable configurations (energizable or non-energizable) are also contemplated, e.g., a hook-shaped configuration (like energizable member 220 (FIG. 2)), ball-shaped configuration, needle-shaped configuration, etc.

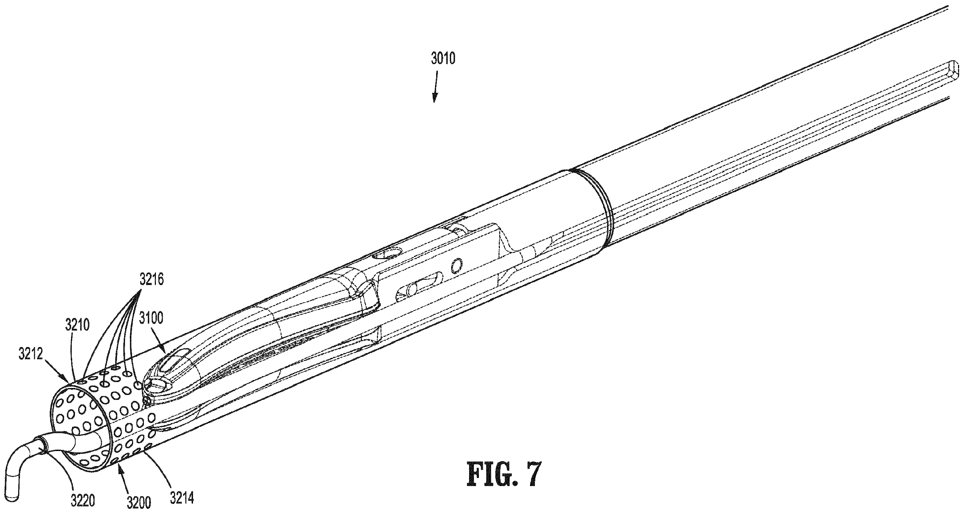

With reference to FIG. 7, another multi-function surgical instrument provided in accordance with the present disclosure is shown generally identified by reference numeral 3010. Instrument 3010 is similar to instrument 10 (FIG. 1) and may include any of the features thereof. Thus, for purposes of brevity, only the differences between instrument 3010 and instrument 10 (FIG. 1) are described in detail below while similarities are summarily described or omitted entirely.

Deployable assembly 3200 of instrument 3010 includes a sheath 3210 and an energizable member 3220. Energizable member 3220 may be configured similarly as energizable member 220 (FIG. 2), energizable member 2200 (FIG. 6), any other suitable energizable member, a mechanical component without energization such as those detailed herein, or may be omitted entirely. Sheath 3210 of deployable assembly 3200 includes a distal portion 3212 defining a plurality of apertures 3216 through cylindrical side wall 3214 thereof. Apertures 3216 permit passage of fluid therethrough into and out of the interior of sheath 3210. Sheath 3210 of deployable assembly 3200 is coupled to a suction and/or irrigation source (see source 400 (FIG. 1)) to enable the delivery of fluid to and/or withdrawal of fluid from the surgical site via sheath 3210, e.g., via the open distal end of sheath 3210 and/or via apertures 3216. Distal portion 3212 of sheath 3210, which includes apertures 3216, may be defined as the portion of sheath 3210 that extends distally from end effector assembly 3100 in the extended position of deployable assembly 3200, or may extend further proximally about sheath 3210. Apertures 3216 may be arranged in any suitable pattern, may extend annularly about the entire circumference of sheath 3210 or just a portion thereof, e.g., apertures 3216 may extend about 90 degrees, 180 degrees, or 270 degrees of the circumference of sheath 3210, or may intermittently be disposed about sheath 3210, e.g., to deliver or suction fluid from opposed lateral sides of sheath 3210.

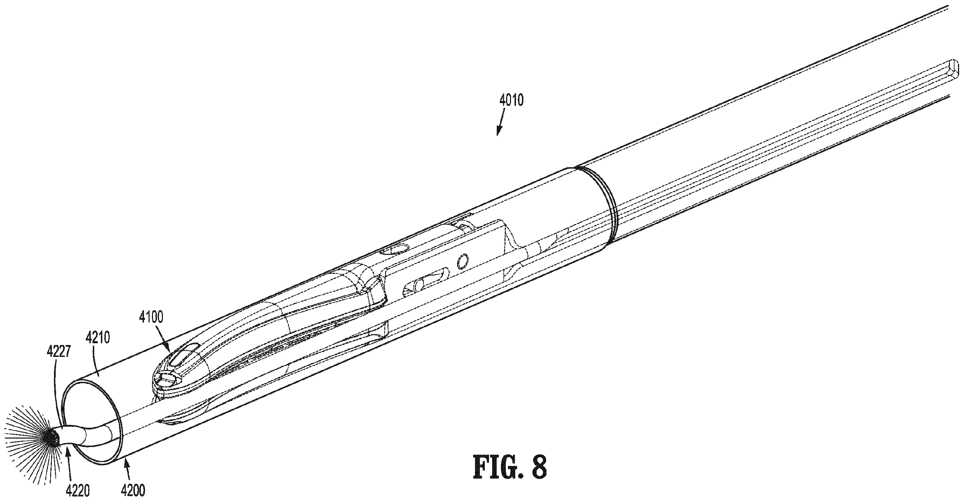

As illustrated in FIG. 8, another multi-function surgical instrument provided in accordance with the present disclosure is shown generally identified by reference numeral 4010. Instrument 4010 is similar to instrument 10 (FIG. 1) and may include any of the features thereof. Thus, for purposes of brevity, only the differences between instrument 4010 and instrument 10 (FIG. 1) are described in detail below while similarities are summarily described or omitted entirely.

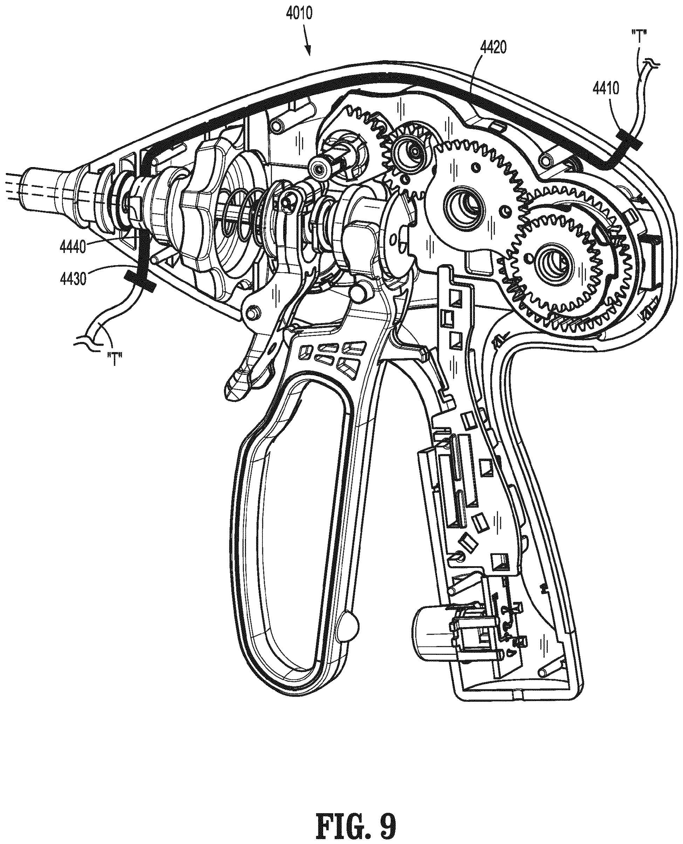

Deployable assembly 4200 of instrument 4010 includes a sheath 4210 and a fluid jet member 4220. Sheath 4210 may be configured similarly as any of the other sheaths detailed herein or in any other suitable manner. In other configurations, sheath 4210 is omitted entirely. With additional reference to FIG. 9, sheath 4210, where provided, is coupled to a suction source (see source 400 (FIG. 1)) via first internal fluid line 4420, first fluid port 4410, and tubing "T" to enable the withdrawal of fluid from a surgical site into the open distal end of sheath 4210 and through the interior of sheath 4210. Suction may be applied through sheath 4210 in the retracted position of deployable assembly 4200, in the extended position of deployable assembly 4200, or in both the retracted and extended positions of deployable assembly 4200. Thus, suction may be provided before, during, and/or after use of instrument 10 in the first mode of operation.

Continuing with reference to FIGS. 8 and 9, fluid jet member 4220 is movable together with sheath 4210 and relative to end effector assembly 4100 between a retracted position, wherein distal nozzle portion 4227 of fluid jet member 4220 is positioned more-proximally, and an extended position, wherein distal nozzle portion 4227 of fluid jet member 4220 extends distally from end effector assembly 2100 to facilitate supplying fluid to a surgical site. Fluid jet member 4220 is configured as fluid tube and may include distal nozzle portion 4227 to facilitate delivering fluid in the form of a jet stream, e.g., to clear blood, debris, etc. from the surgical site, to blast debris or tissue with the surgical site, and/or to cut through delicate tissue. As an alternative to a fluid jet, member 4220 may deliver fluid in any other suitable manner.

Fluid jet member 4220 is coupled to a fluid source (see source 400 (FIG. 1) or a separate source) via second internal fluid line 4440, second fluid port 4430, and tubing "T" to enable the supply of fluid to fluid jet member 4220 for application to a surgical site in the form of a fluid jet stream. Fluid jet member 4220 and/or the source may be configured to provide a variable, controllable pressure and/or flow rate of the fluid jet stream. The fluid may be water, saline, air, CO₂, medicament, cryogenic fluid, a surgical adhesive, or other suitable liquid or gas. Fluid may be supplied from fluid jet member 4220 in the retracted position of deployable assembly 4200, in the extended position of deployable assembly 4200, or in both the retracted and extended positions of deployable assembly 4200. Thus, fluid may be provided before, during, and/or after use of instrument 10 in the first mode of operation.

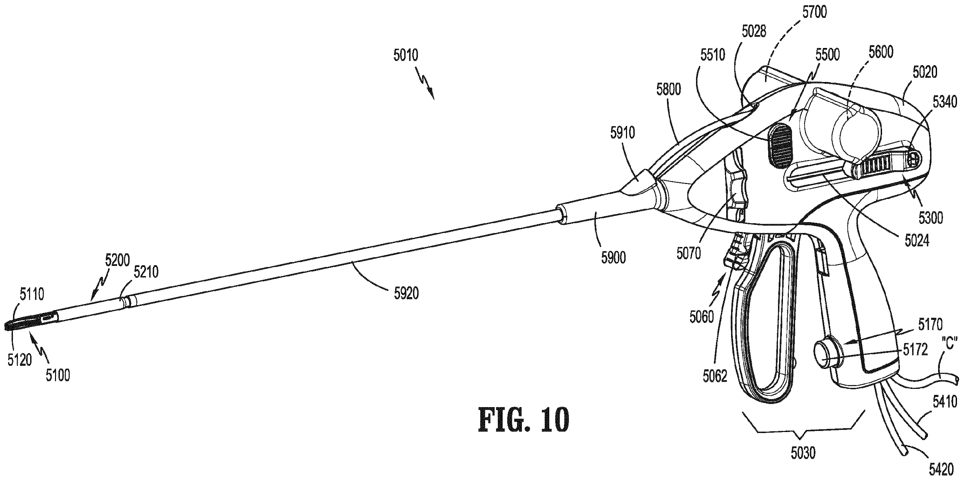

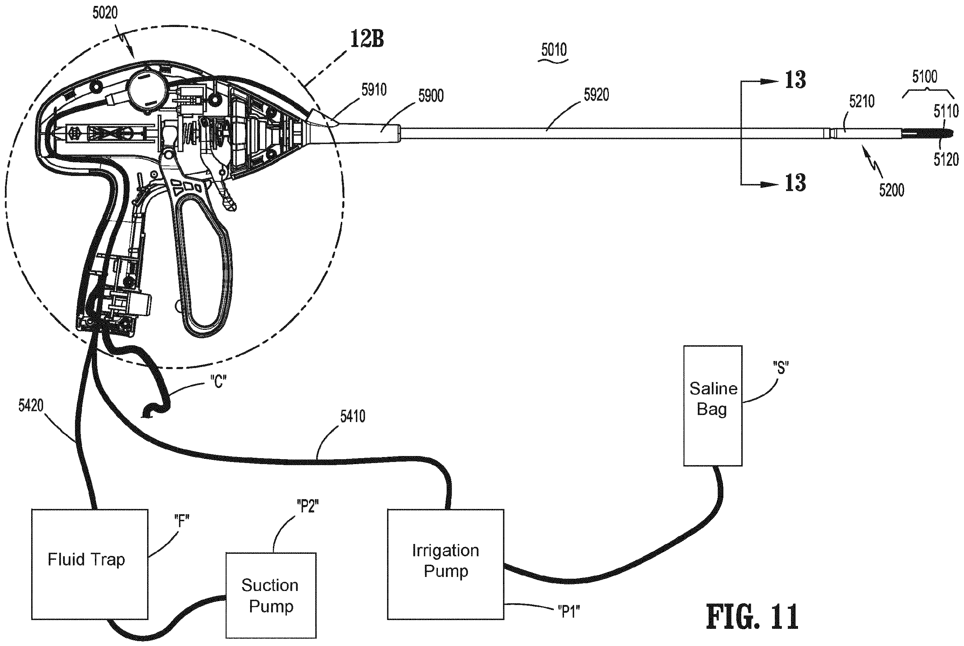

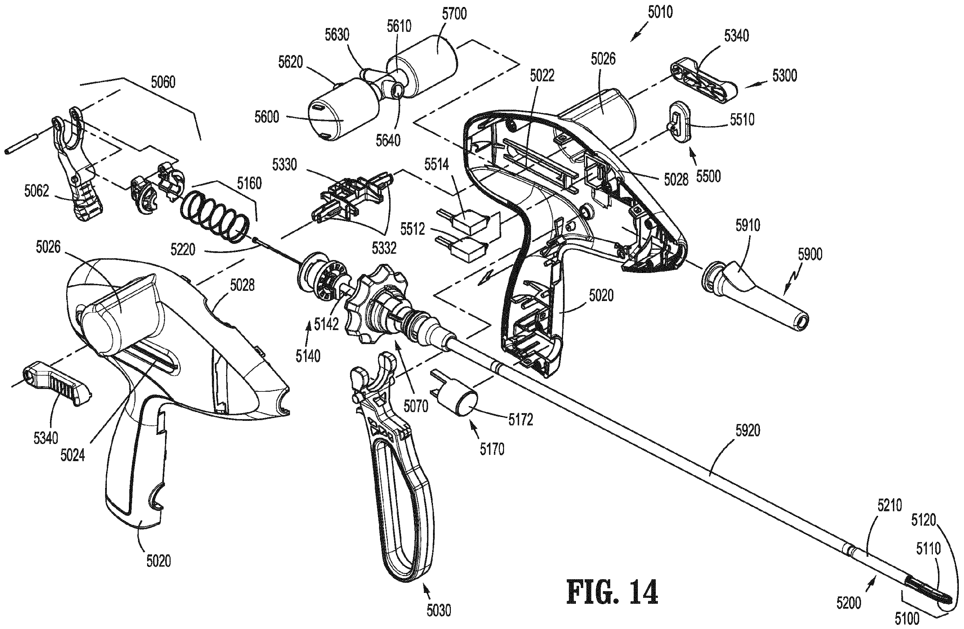

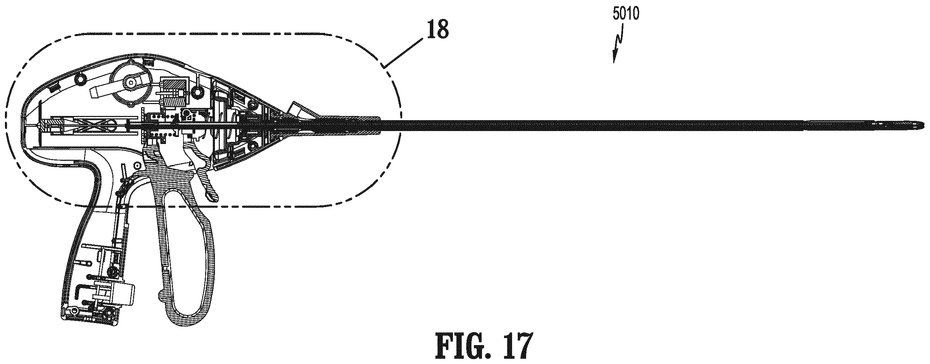

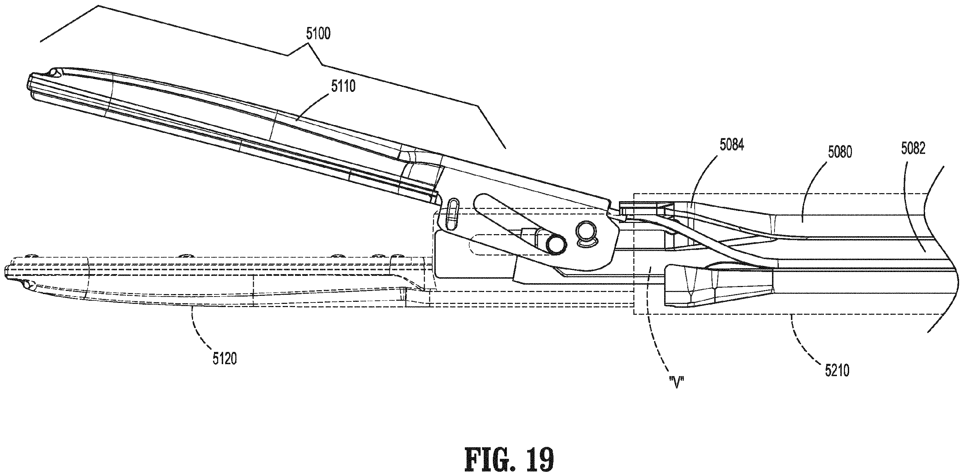

Referring generally to FIGS. 10-19, another multi-function surgical instrument 5010 provided in accordance with the present disclosure is configured to operate in at least a first mode, e.g., for grasping tissue, treating (e.g., sealing) grasped tissue with bipolar energy, and/or mechanically dissecting grasped tissue, and further includes suction and/or irrigation capability for use before, during, and/or after the first mode of operation. Multi-function surgical instrument 5010 may additionally or alternatively be configured to operate in a second mode, e.g., for treating tissue and/or electrically/electromechanically dissecting tissue with monopolar, thermal, microwave, or other suitable energy, or any other suitable mode facilitating tissue treatment. For purposes of brevity, only differences between multi-function surgical instrument 5010 and the above-detailed multi-function surgical instruments 10, 1010, 2010, 3010, 4010 (FIGS. 1, 5, 6, 7, 8, respectively) are described in detail below while similarities are summarily described or omitted entirely. To the extent consistent, any of the features of any of the multi-function surgical instruments 10, 1010, 2010, 3010, 4010, 5010 (FIGS. 1, 5, 6, 7, 8, 10, respectively) detailed herein may be utilized in connection with any or all of the other multi-function surgical instruments 10, 1010, 2010, 3010, 4010, 5010 (FIGS. 1, 5, 6, 7, 8, 10, respectively) detailed in any suitable combination.

With reference to FIGS. 10-14, multi-function surgical instrument 5010 includes a housing 5020, a handle assembly 5030, a trigger assembly 5060, a rotation assembly 5070, an elongated shaft 5080, an end effector assembly 5100, a drive assembly 5140, a knife assembly 5160, an activation assembly 5170, a deployable assembly 5200, a deployment and retraction mechanism 5300, a selector assembly 5500, and first and second valve assemblies 5600, 5700.

Instrument 5010 also includes an electrosurgical cable "C" that connects instrument 5010 to a generator (not shown) or other suitable power source, an inflow tube 5410 associated with first valve assembly 5600 and adapted to connect to a source of fluid, e.g., a saline bag "S," and a pump, e.g., an irrigation pump "PI," and an outflow tube 5420 associated with second valve assembly 5700 and adapted to connect to a collection vessel, e.g., a fluid trap "F," and a pump, e.g., suction pump "P2." More specifically, inflow and outflow tubes 5410, 5420, respectively, extend into housing 5020 wherein inflow and outflow tubes 5410, 5420 couple to first and second valve assemblies 5600, 5700, respectively, which, in turn, connect to a common tube 5800 that connects with at least a portion of the interior of sheath 5210 of deployable assembly 5200 to enable the delivery of fluid to and/or withdrawal of fluid from the interior of sheath 5210. Irrigation through sheath 5210 is thus achieved by activating irrigation pump "PI" and opening first valve assembly 5600 to pump fluid, e.g., saline from saline bag "S," through inflow tubing 5410, first valve assembly 5600, common tube 5800, and the interior of sheath 5210, and out the open distal end of sheath 5210 and into a surgical site. Suction through sheath 5210, on the other hand, is achieved via activating suction pump "P2" and opening second valve assembly 5700 to establish suction within the interior of sheath 5210, through common tube 5800, through second valve assembly 5700, and through outflow tube 5420 to fluid trap "F."

When first valve assembly 5600 is open, corresponding to the irrigation mode, second valve assembly 5700 is closed, and when second valve assembly 5700 is open, corresponding to the suction mode, first valve assembly 5600 is closed. Selector assembly 5500, disposed on housing 5020, may include a toggle button 5510 or other suitable button(s), e.g., slider buttons, individual buttons, rotational buttons, etc., to enable switching between the irrigation mode and the suction mode. That is, when toggle button 5510 is moved to a first position, corresponding to the irrigation mode, first valve assembly 5600 is opened and second valve assembly 5700 is closed. When toggle button 5510 is moved to a second position, corresponding to the suction mode, second valve assembly 5700 is opened and first valve assembly 5600 is closed. Toggle button 5510 may further include a neutral position wherein both first and second valve assemblies 5600, 5700, respectively, are closed. Toggle button 5510 may interact with underlying electrical switches 5512, 5514 connected to first and second valve assemblies 5600, 5700, respectively, such that valve assemblies 5600, 5700 are electrically controlled to open and close based upon the position of toggle button 5510. In such configurations, valve assemblies 5600, 5700 may include solenoid valves or other suitable electrically-controlled valves. In configurations where electrically-controlled valves are employed, a battery (not shown) may be disposed within housing 5020 for powering the same or the electrically-controlled valves may be coupled to an external source of energy, e.g., via connection of cable "C" to a generator. Alternatively, moving toggle button 5510 between its various positions may mechanically operate valve assemblies 5600, 5700 such as, for example, functioning as a stopcock for the valve assemblies 5600, 5700. Other non-electrically-controlled valves such as, for example, trumpet valves, may also be utilized.

Manipulating toggle button 5510 between the first, second, and neutral positions may also automatically start or stop pumps "PI" and "P2." For example, when toggle button 5510 is moved to the first position, underlying electrical switch 5512 may signal pump "PI" to be activated to initiate irrigation and, when toggle button 5510 is moved from the first position to a different position, underlying electrical switch 5512 may signal pump "PI" to be deactivated to stop irrigation. Likewise, when toggle button 5510 is moved to the second position, underlying electrical switch 5514 may signal pump "P2" to be activated to initiate suction and, when toggle button 5510 is moved from the second position to a different position, underlying electrical switch 5514 may signal pump "P2" to be deactivated to stop suction. However, it is also contemplated that, in some configurations, the starting and/or stopping of irrigation and/or suction is not effected immediately upon receipt of the signals. For example, irrigation and/or suction may continue for a pre-determined time, e.g., 5 second, 10 seconds, etc., after toggle button 5510 is moved out of the corresponding position to enable the fluid lines to be cleared, e.g., to prevent backflow, overflow, etc., due to the fact that the suction and irrigation flow paths share common tube 5800 and the interior of sheath 5210.

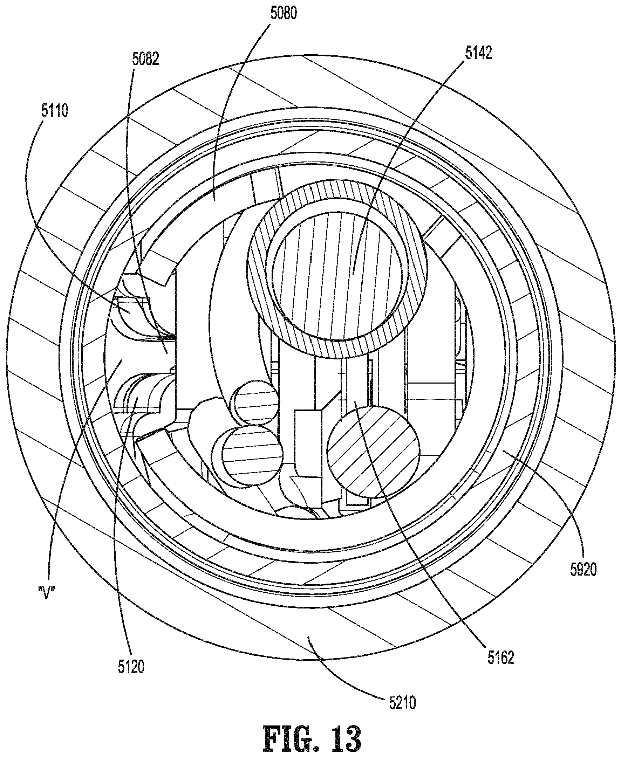

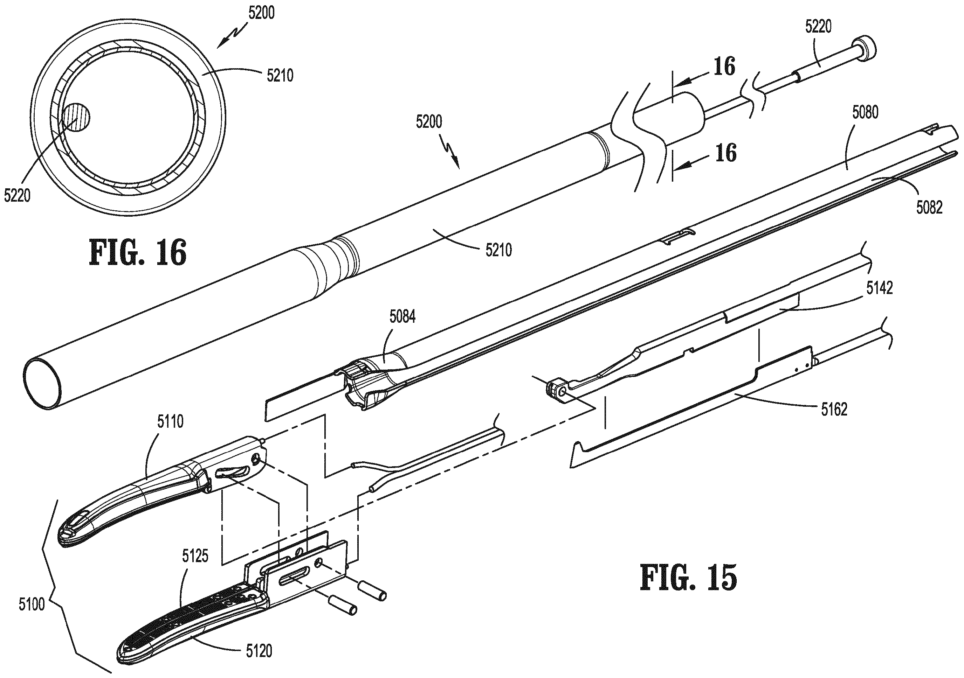

Referring also to FIGS. 15-19, elongated shaft 5080 is supported within housing 5020, extends distally from housing 5020, and supports end effector assembly 5100 at a distal end thereof. Elongated shaft 5080 may define a C-shaped configuration along at least a portion of a length thereof such that a side-facing opening 5082 communicating with the interior of elongated shaft 5080 extends the at least a portion of the length thereof. This side-facing opening 5082 provides a greater unobstructed internal volume "V" within sheath 5210 for the suction and/or irrigation of fluid therethrough through, thus enabling a greater fluid delivery/removal rate. End effector assembly 5100 includes opposing jaw members 5110, 5120 configured to enable grasping of tissue therebetween, e.g., via actuation of drive bar 5142 of drive assembly 5140 in response to actuation of handle assembly 5030, and conducting energy through tissue grasped therebetween for treating tissue, e.g., in response to activation of activation button 5172 of activation assembly 5170. Jaw member 5110, 5120 may define curved configurations wherein jaw members 5110, 5120 curve off a longitudinal axis defined by elongated shaft 5080. In such configurations, the side-facing opening 5082 of elongated shaft 5080 may be disposed on the concave side of jaw members 5110, 5120 such that the void defined on the concave side of jaw members 5110, 5120 provides additional unobstructed internal volume "V" within sheath 5210, directly communicating with the above-detailed volume, to facilitate suction and/or irrigation of fluid therethrough (see FIG. 13). Further, elongated shaft 5080 includes a flared distal end portion 5084 for similar purposes.

Trigger 5062 of trigger assembly 5060 is selectively actuatable relative to housing 5020 from an un-actuated position to an actuated position to thereby actuate knife assembly 5160 to translate knife 5162 of knife assembly 5160 from a retracted position, wherein knife 5162 is disposed proximally of jaw members 5110, 5120, to an extended position, wherein knife 5162 extends at least partially between jaw members 5110, 5120 and through knife channels 5125 (only knife channel 5125 of jaw member 5120 is shown; the knife channel of jaw member 5110 is similar) defined within jaw members 5110, 5120 to cut tissue grasped between jaw members 5110, 5120.

Deployable assembly 5200 includes a sheath 5210 and an actuation rod 5220. Sheath 5210 may be at least partially insulative, although other configurations are also contemplated. Sheath 5210 may include one or more sheath layers secured to one another to form sheath 5210. Sheath 5210 is movable relative to end effector assembly 5100 between a retracted position, wherein sheath 5210 is disposed proximally of end effector assembly 5100, and an extended position, wherein sheath 5210 is substantially disposed about end effector assembly 5100 and/or extends distally therefrom. Actuation rod 5220 is engaged with sheath 5210 within sheath 5210 at a proximal end portion of sheath 5210 such that actuation rod 5220 moves to thereby move sheath 5210 between the retracted and extended positions. A proximal end portion of actuation rod 5220 is engaged with a slider body 5330 of deployment and retraction mechanism 5300. Slider body 5330 is disposed within housing 5020 and, more specifically, is slidably engaged within longitudinal tracks 5022 defined on the interior surface of housing 5020 to confine slider body 5230 to longitudinal translation within and relative to housing 5020. Slider body 5330 includes wings 5332 extending outwardly from each side thereof and through slots 5024 defined within opposing sides of housing 5020. Slide knobs 5340 are mounted on wings 5332 on opposing exterior sides of housing 5020. Either or both of the slide knobs 5340 is slidable along housing 5020 from a more-proximal position to a more-distal position to thereby translate slider body 5330 within housing 5020 between a more-proximal position and a more-distal position. Movement of slider body 5330 between the more-proximal position and the more-distal position, in turn, moves actuation rod 5220 and, thus, sheath 5210 between the retracted and deployed positions. Other suitable deployment mechanisms are also contemplated.

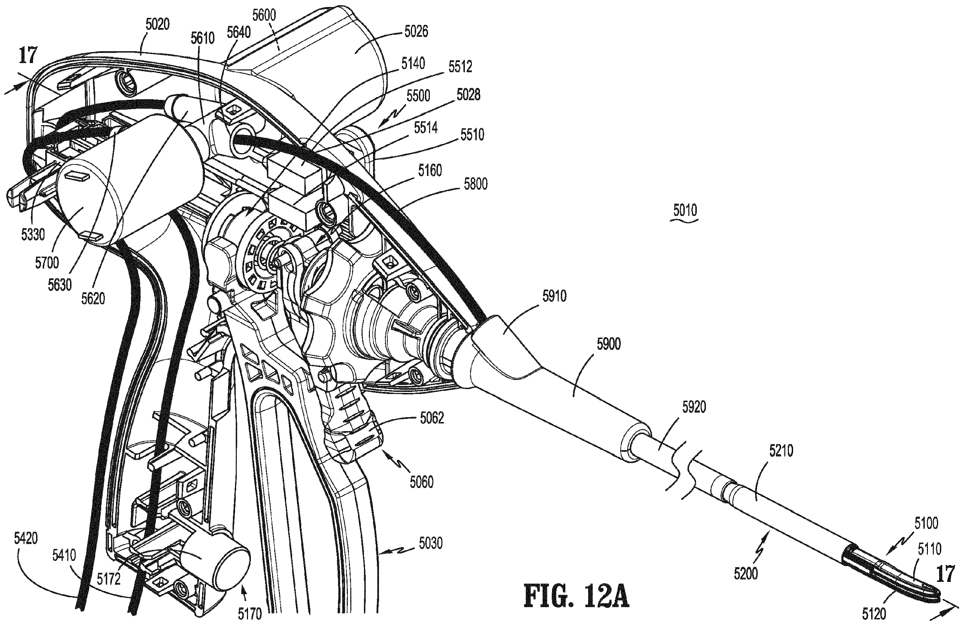

With reference to FIGS. 12A, 12B, 14, and 18, as noted above, inflow and outflow tubes 5410, 5420 extend into housing 5020 and couple with first and second valve assemblies 5600, 5700, respectively, which, in turn, couple with common tube 5800. First and second valve assemblies 5600, 5700, more specifically, share a common manifold 5610 having first and second ports 5620, 5630 for connection to inflow and outflow tubes 5410, 5420, respectively, and a common port 5640 for connection to common tube 5800. The opening and closing of first and second valve assemblies 5600, 5700, as detailed above, thus controls the flow of fluid between inflow and outflow tubes 5410, 5420 and common tube 5800. First and second valve assemblies 5600, 5700 are at least partially seated within pockets 5026 defined within and protruding outwardly from opposing sides of housing 5020, with common manifold 5610 extending transversely through housing 5020 between first and second valve assemblies 5600, 5700.

Common tube 5800 is connected to common port 5640 of common manifold 5610 within housing 5020 and exits housing 5020 proximally of elongated shaft 5080 via an aperture 5028 defined within housing 5020. Upon exiting housing 5020, common tube 5800 extends distally along housing 5020 and distally from housing 5020 wherein common tube 5800 extends into a port 5910 defined within a fixed collar 5900 extending distally from housing 5020 about a portion of sheath 5210. In configurations, fixed collar 5900 extends less than 50% of a length of elongated shaft 5080, less than 35% of the length of elongated shaft 5080, or less than 20% of the length of elongated shaft 5080. A fixed outer tube 5920 engaged with and extending distally from fixed collar 5900 about sheath 5210 may extend, in configurations, at least 50% of an exposed length of sheath 5210, at least 60% of an exposed length of sheath 5210, or at least 70% of an exposed length of sheath 5210. Other configurations are also contemplated.

As noted above, actuation rod 5220 is engaged within sheath 5210 at a proximal end portion of sheath 5210. The open proximal end of sheath 5210 and the engagement between actuation rod 5220 and sheath 5210 may be disposed within fixed collar 5900. Alternatively, the open proximal end of sheath 5210 and the engagement between actuation rod 5220 and sheath 5210 may be within housing 5020 proximally of fixed collar 5900 and sheath 5210. In such configurations, sheath 5210 may include one or more openings that are disposed within fixed collar 5900. In either of the above configurations, a plug 5930 is sealingly engaged within fixed collar 5900 and sealingly coupled about sheath 5210 and/or elongated shaft 5080 while permitting sliding of sheath 5210 relative thereto between the retracted and deployed positions. Plug 5930 defines one or more annularly-arranged openings 5940 that communicate with the open proximal end of sheath 5210 and/or the one or more openings of sheath 5210. In this manner, fluid pumped distally though common tube 5800 and into port 5910 of fixed collar 5900 is directed through openings 5940 of plug 5930, into the interior of sheath 5210, and distally through sheath 5210 out the open distal end thereof to irrigate a surgical site. Likewise, in a suction mode, fluid is suctioned proximally from the open distal end of sheath 5210 through the interior of sheath 5210, into plug 5930, through openings 5940 of plug 5930, into port 5910, and to common tube 5800.

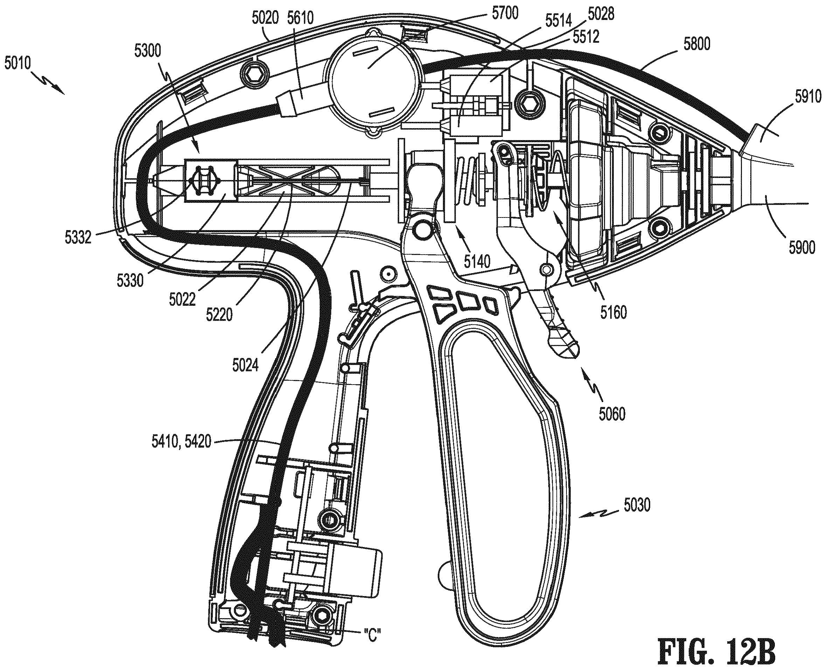

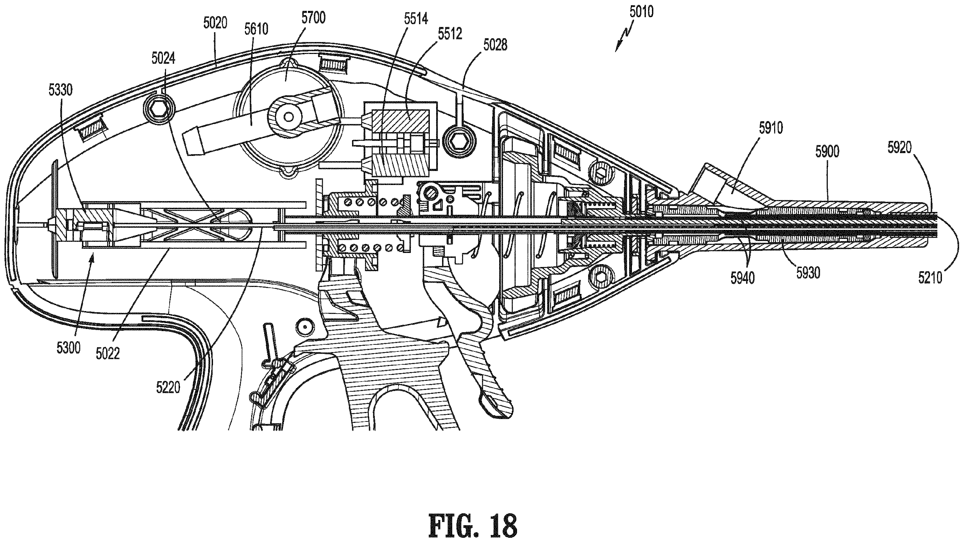

Referring to FIGS. 12A, 12B, and 18, first and second valve assemblies 5600, 5700 are positioned in close proximity (not withstanding functional constraints and/or ergonomic considerations) to plug 5930 and, thus, to the interior of sheath 5210 and the open distal end thereof, through which fluid is irrigated or suctioned. By minimizing the distance between first and second valve assemblies 5600, 5700 and the open distal end of sheath 5210, the response times for activating/deactivating suction and irrigation as well as the amount of backflow can be reduced, thus improving control.

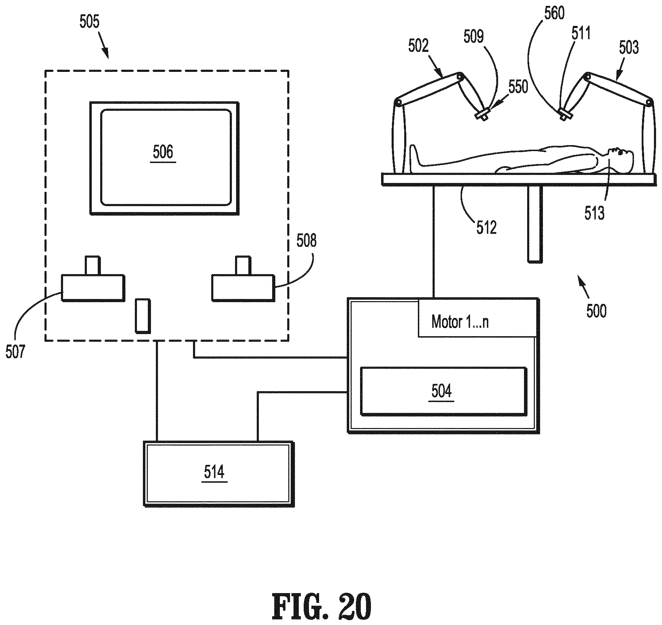

Turning to FIG. 20, as opposed to handheld, manual manipulation and operation, the various aspects and features disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical system may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the aspects and features described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

Continuing with reference to FIG. 20, one exemplary implementation of a robotic surgical system is shown generally identified by reference numeral 500. Aspects and features of robotic surgical system 500 not germane to the understanding of the present disclosure are omitted to avoid obscuring the aspects and features of the present disclosure in unnecessary detail.

Robotic surgical system 500 includes a plurality of robot arms 502, 503; a control device 504; and an operating console 505 coupled with control device 504. Operating console 505 may include a display device 506, which may be set up in particular to display three dimensional images; and manual input devices 507, 508, by means of which a surgeon may be able to telemanipulate robot arms 502, 503 in a first operating mode. Robotic surgical system 500 may be configured for use on a patient 513 lying on a patient table 512 to be treated in a minimally invasive manner. Robotic surgical system 500 may further include a database 514, in particular, coupled to control device 504, in which are stored, for example, pre-operative data from patient 513 and/or anatomical atlases.

Each of the robot arms 502, 503 may include a plurality of members, which are connected through joints, and an attaching device 509, 511, to which may be attached (directly or indirectly via intermediate structures), a surgical instrument 550, for example, any of the surgical instrument detailed hereinabove adapted for use in a robotic surgical system (e.g., replacing any manual grasping or control features with robotic attachments and inputs), and a surgical instrument 560. Surgical instrument 560 may be any suitable surgical instrument, e.g., an endoscopic camera, other surgical tool, etc. Robot arms 502, 503 and surgical instruments 550, 560 may be driven by electric drives, e.g., motors, that are connected to control device 504. Control device 504 (e.g., a computer) may be configured to activate the motors, in particular by means of a computer program, in such a way that robot arms 502, 503, their attaching devices 509, 511, and surgical instruments 550, 560 execute a desired movement and/or function according to a corresponding input from manual input devices 507, 508, respectively. Control device 504 may also be configured in such a way that it regulates the movement of robot arms 502, 503 and/or of the motors.

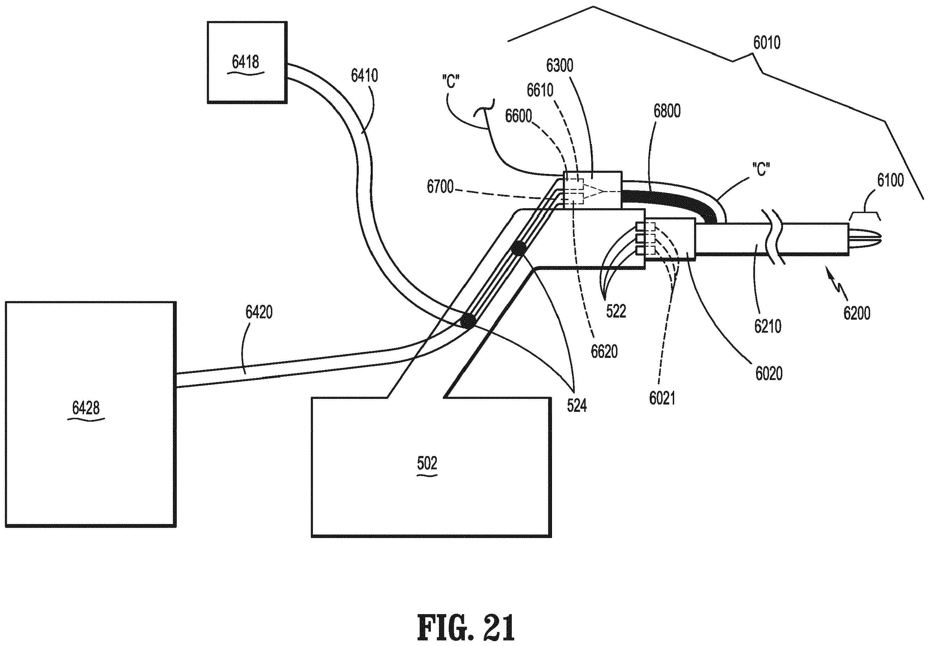

With reference to FIG. 21, a multi-function surgical instrument 6010 configured for use with robotic surgical system 500 (FIG. 20) is shown. Surgical instrument 6010, more specifically, is configured for mounting on a robot arm, e.g., robot arm 502, of surgical system 500 (FIG. 20). Surgical instrument 6010 may be similar to and include any of the features of any of the other multi-function surgical instruments detailed herein and, thus, only differences therebetween are described in detail below while similarities are summarily described or omitted entirely.

Surgical instrument 6010 includes a housing 6020 configured to operably mount to robot arm 502, an elongated shaft (not explicitly shown) extending distally from housing 6020, an end effector assembly 6100 disposed at a distal end of the elongated shaft, and a deployable assembly 6200 including a sheath 6210 selectively deployable relative to end effector assembly 6100 between a retracted position and a deployed position. Housing 6020 includes a fluid control box 6300 configured to operably mount on robot arm 502 separately or in conjunction with the mounting of the main body of housing 6020. Alternatively, fluid control box 6300 may be disposed on or within the main body of housing 6020 and, thus, may be attachable to robot arm 502 via the attachment of housing 6020 thereto.

Housing 6020 includes a plurality of inputs 6021 operably coupled thereto. Inputs 6021 are configured to operably connect with corresponding outputs 522 of robot arm 502 upon mounting of housing 6020 thereon. One or more of inputs 6021 is operably coupled to each of a drive assembly (not shown), knife assembly (not shown), and deployment and retraction mechanism (not shown) within housing 6020, such that, instead of manual actuation of these various assemblies and mechanisms, outputs 522 of robot arm 502 may be selectively actuated to operate these various assemblies and mechanisms. An electrical cable "C" connected to fluid control box 6300 or, alternatively, housing 6020 is configured to connect instrument 6010 to a generator (not shown) to enable the supply of electrosurgical energy to end effector assembly 6100.

Fluid control box 6300 of housing 6020 houses first and second valve assemblies 6600, 6700 and, in configurations where valve assemblies 6600, 6700 include electrically-controlled valves, e.g., solenoid valves, may likewise be configured to connect to a source of energy, e.g., via cable "C." Valves 6610, 6710 of valve assemblies 6600, 6700, respectively, couple with inflow and outflow tubes 6410, 6420, respectively, at first ends thereof, and with common tube 6800 at second ends thereof. Common tube 6800, in turn, connects with at least a portion of the interior of sheath 6210 of deployable assembly 6200 to enable the delivery of fluid to and/or withdrawal of fluid from the interior of sheath 6210. Inflow tube 6410 is adapted to connect to a fluid source and pump 6418 (which may be integrated or separate), while outflow tube 6420 is adapted to connect to a fluid receptacle and pump 6428 (which likewise may be integrated or separate). Robot arm 502 may include tube guides 524, e.g., clips, hooks, posts, recesses, channels, etc., to guide inflow and outflow tubes 6410, 6420 at least partially therealong to facilitate routing to fluid source and pump 6418 and fluid receptacle and pump 6428, respectively. Swivel joints, connectors, etc. formed along inflow and/or outflow tubes 6410, 6420 and/or any other suitable tube routing and movement features may be provided to inhibit interference with movement of robot arm 502 while also facilitating the routing of inflow and outflow tubes 6410, 6420 are also contemplated.

Instrument 6010 and/or another operably coupled device, e.g., surgical instrument 560 (FIG. 20), may, in some configurations, incorporate a sensor, e.g., a flow sensor, a fluid sensor, a pressure sensor, a visual sensor (e.g., camera), etc., to enable automatic starting and stopping of suction and/or irrigation based on a detected presence and/or amount of fluid in a surgical site, a clarity of the surgical site, a presences and/or amount of smoke in the surgical site, etc. With respect to monitoring fluid, for example, the amount of fluid added and extracted can be monitored to provide alerts if the fluid added versus fluid extracted balance is outside a threshold, e.g., thus indicating there is significant blood loss, too much fluid in the surgical site, etc. Additional or alterative determinations made based on sensed fluid in and out can be utilized to facilitate interoperative fluid management and/or to maintain appropriate fluid levels based upon a tissue treatment to be performed, e.g., an appropriate fluid level to minimize steam and maximize tissue sealing capability when operating in the first tissue treatment mode of operation.

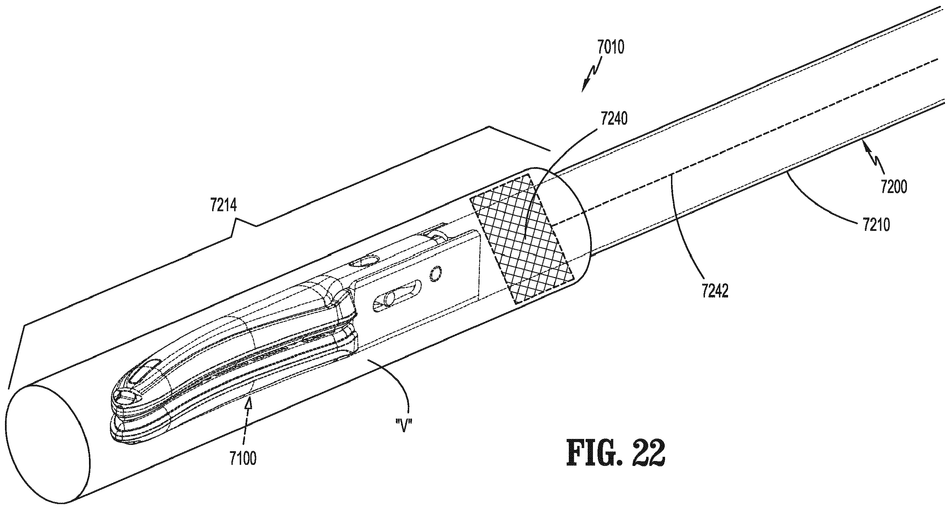

Referring to FIG. 22, a distal end portion of another multi-function surgical instrument 7010 in accordance with the present disclosure is shown. Surgical instrument 7010 may be similar to and include any of the features of any of the other multi-function surgical instruments detailed herein and, thus, only differences therebetween are described in detail below while similarities are summarily described or omitted entirely.

Deployable assembly 7200 of instrument 7010 includes a sheath 7210 movable relative to end effector assembly 7100 between a retracted position, wherein sheath 7210 is disposed proximally of end effector assembly 7100, and an extended position (as shown), wherein sheath 7210 is substantially disposed about end effector assembly 7100. Deployable assembly 7200 of instrument 7010 further includes an expandable structure 7240 disposed within sheath 7210 and movable therewith. Alternatively, expandable structure 7240 may be disposed on end effector assembly 7100 and fixed relative thereto such that sheath 7210 is movable relative to expandable structure 7240. In either configuration, expandable structure 7240 may extend along at least a portion of an expandable distal section 7214 of sheath 7210. Expandable distal section 7214, in configurations, defines a length equal to or greater than end effector assembly 7100. Expandable structure 7240 may include an inflatable bladder, expandable scaffold, shape-memory material, spring-biased expander, and/or other suitable structure(s) to enable selective expansion of expandable structure 7240 to thereby expand expandable distal section 7214 of sheath 7210. Expandable distal section 7214 may be configured to expand uniformly, e.g., to maintain a generally cylindrical configuration, disproportionally, e.g., to define a cone-shaped expanded configuration, irregularly, or in any other suitable manner. Expandable distal section 7214 may be formed from a flexible material, an expandable structure, or in any other suitable manner.

An actuator 7242, e.g., a fluid line, mechanical actuator, etc., is coupled to expandable structure 7240 to enable the selective expansion thereof to thereby expand expandable distal section 7214 of sheath 7210. Actuator 7242 may be independently actuated to expand and/or contract expandable structure 7240 or may be actuated upon deployment and/or retraction of sheath 7210. Expandable structure 7240 may be inhibited from expansion in the retracted position of sheath 7210 or may be expandable in the retracted position of sheath 7210.

Regardless of the particular configuration of expandable distal section 7214 and expandable structure 7240, the expansions thereof establish a greater unobstructed internal volume "V" within expandable distal section 7214 and around end effector assembly 7100, thus facilitating suction and/or irrigation.

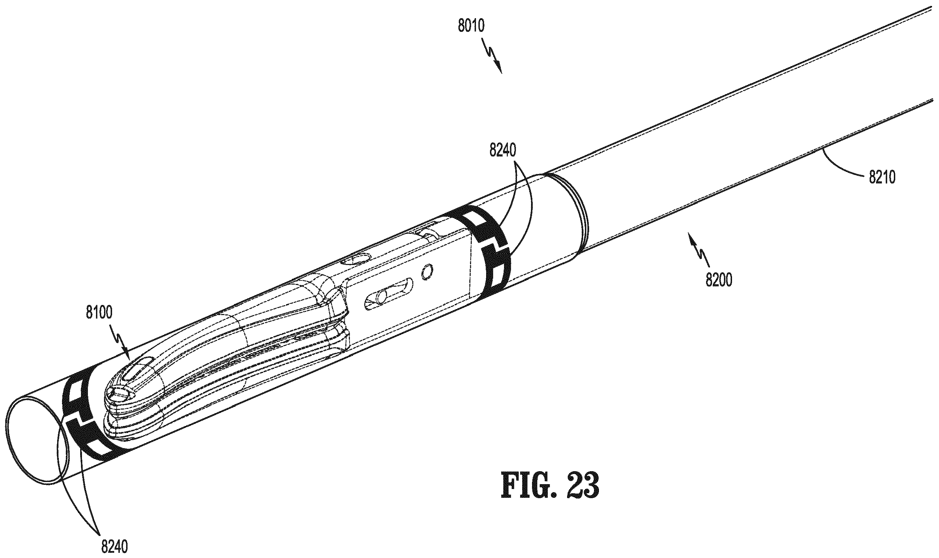

With reference to FIG. 23, a distal end portion of another multi-function surgical instrument 8010 in accordance with the present disclosure is shown. Surgical instrument 8010 may be similar to and include any of the features of any of the other multi-function surgical instruments detailed herein and, thus, only differences therebetween are described in detail below while similarities are summarily described or omitted entirely.

Deployable assembly 8200 of instrument 8010 includes a sheath 8210 movable relative to end effector assembly 8100 between a retracted position, wherein sheath 8210 is disposed proximally of end effector assembly 8100, and an extended position (as shown), wherein sheath 8210 is substantially disposed about end effector assembly 8100. Deployable assembly 8200 of instrument 8010 further includes one or more flow directors 8240 disposed within and coupled to sheath 8210 and movable therewith. Flow directors 8240 may be deployable, e.g., upon deployment of sheath 8210, or may be fixed. As an alternative or in addition to being coupled to sheath 8210, flow director(s) 8240 may be coupled to end effector assembly 8100 and movable relative to sheath 8210. In either configuration, the one or more flow directors 8240 may include curved, angled, or otherwise configured plates, channels, guides, or other suitable structures, positioned to direct the flow of fluid within sheath 8210 around end effector assembly 8100, thus facilitating suction and/or irrigation. More specifically, one or more flow directors 8240 may be disposed within sheath 8210 distally of end effector assembly 8100 to direct the flow of fluid suctioned through sheath 8210 and/or one or more flow directors 8240 may be disposed within sheath 8210 proximally of end effector assembly 8100 to direct the flow of irrigation fluid through sheath 8210.

From the foregoing and with reference to the various drawings, those skilled in the art will appreciate that certain modifications can be made to the present disclosure without departing from the scope of the same. While several aspects and features of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular configurations. Those skilled in the art will envision other modifications within the scope and spirit of the claims appended hereto.

The invention may be described by reference to the following numbered paragraphs:-
1. A surgical instrument, comprising:
   an elongated shaft;
   an end effector assembly supported at a distal end portion of the elongated shaft;
   a sheath disposed about the elongated shaft and movable relative to the elongated shaft and the end effector assembly between a retracted position, wherein the sheath is positioned proximally of the end effector assembly, and an extended position, wherein the sheath is disposed about the end effector assembly;
   a housing disposed at a proximal end portion of the elongated shaft;
   an inflow tube extending into the housing;
   an outflow tube extending into the housing;
   a common tube extending into the housing, the common tube fluidly coupled to an interior of the sheath and configured to supply fluid thereto and remove fluid therefrom;
   a manifold disposed within the housing, the manifold defining an inflow port fluidly coupled to the inflow tube, an outflow port fluidly coupled to the outflow tube, and a common tube port fluidly coupled to the common tube; and
   first and second valve assemblies disposed within the housing and operably coupled to the manifold, the first valve assembly configured to selectively control a flow of fluid from the inflow tube to the interior of the sheath, the second valve assembly configured to selectively control a flow of fluid from the interior of the sheath to the outflow tube.
2. The surgical instrument according to paragraph 1, wherein each of the first and second valve assemblies includes a solenoid valve.
3. The surgical instrument according to paragraph 2, further comprising a selector assembly including first and second switches configured to selectively operate the solenoid valves of the first and second valve assemblies, respectively.
4. The surgical instrument according to paragraph 3, further comprising a toggle switch disposed on the housing and configured to selectively actuate the first and seconds switches.
5. The surgical instrument according to paragraph 1, wherein the elongated shaft defines a C-shaped configuration and includes a side-facing opening defined therein along a portion of a length thereof.
6. The surgical instrument according to paragraph 5, wherein at least one drive component associated with the end effector assembly extends through the elongated shaft.
7. The surgical instrument according to paragraph 1, wherein the elongated shaft defines a flared distal portion.
8. The surgical instrument according to paragraph 1, wherein the sheath includes an expandable distal portion.
9. The surgical instrument according to paragraph 8, further comprising an expandable structure disposed within the expandable distal portion of the sheath and configured to selectively expand the expandable distal portion of the sheath.
10. The surgical instrument according to paragraph 1, further comprising at least one flow director disposed within the sheath adjacent the end effector assembly, the at least one flow director configured to direct a flow of fluid through the sheath around the end effector assembly.
11. A robotic surgical system, comprising:
   a robotic arm including a plurality of tube guides disposed thereon; and
   a robotic surgical instrument configured to releasably engage the robotic arm, the robotic surgical instrument including:
      a housing;
      an elongated shaft extending distally from the housing;
      an end effector assembly supported at a distal end portion of the elongated shaft;
      a sheath disposed about the elongated shaft and movable relative to the elongated shaft and the end effector assembly between a retracted position, wherein the sheath is positioned proximally of the end effector assembly, and an extended position, wherein the sheath is disposed about the end effector assembly;
      an inflow tube routed through at least one of the plurality of tube guides and extending into the housing;
      an outflow tube routed through at least one of the plurality of tube guides and extending into the housing; and
      first and second valve assemblies disposed within the housing and operably coupled to the inflow and outflow tubes, respectively, the first valve assembly configured to selectively control a flow of fluid from the inflow tube to the interior of the sheath, the second valve assembly configured to selectively control a flow of fluid from the interior of the sheath to the outflow tube.
12. The surgical system according to paragraph 11, wherein the robotic surgical instrument further includes:
   a common tube extending into the housing, the common tube fluidly coupled to an interior of the sheath and configured to supply fluid thereto and remove fluid therefrom; and
   a manifold disposed within the housing, the manifold defining an inflow port fluidly coupled to the inflow tube, and outflow port fluidly coupled to the outflow tube, and a common tube port fluidly coupled to the common tube.
13. The surgical system according to paragraph 11, wherein each of the first and second valve assemblies includes a solenoid valve.
14. The surgical system according to paragraph 13, further comprising first and second switches disposed within the housing and configured to selectively operate the solenoid valves of the first and second valve assemblies, respectively.
15. The surgical system according to paragraph 11, wherein the housing includes a main housing body and a fluid control box, the first and second valve assemblies disposed within the fluid control box.
16. The surgical system according to paragraph 11, wherein the elongated shaft defines a C-shaped configuration and includes a side-facing opening defined therein along a portion of a length thereof.
17. The surgical system according to paragraph 16, wherein at least one drive component associated with the end effector assembly extends through the elongated shaft.
18. The surgical system according to paragraph 11, wherein the elongated shaft defines a flared distal portion.
19. The surgical system according to paragraph 11, wherein the sheath includes an expandable distal portion and wherein an expandable structure is disposed within the expandable distal portion, the expandable structure configured to selectively expand the expandable distal portion of the sheath.
20. The surgical system according to paragraph 11, further comprising at least one flow director disposed within the sheath adjacent the end effector assembly, the at least one flow director configured to direct a flow of fluid through the sheath around the end effector assembly.

## Claims

1. A surgical instrument, comprising:
an elongated shaft;
an end effector assembly supported at a distal end portion of the elongated shaft;
a sheath disposed about the elongated shaft and movable relative to the elongated shaft and the end effector assembly between a retracted position, wherein the sheath is positioned proximally of the end effector assembly, and an extended position, wherein the sheath is disposed about the end effector assembly;
a housing disposed at a proximal end portion of the elongated shaft;
an inflow tube extending into the housing;
an outflow tube extending into the housing;
a common tube extending into the housing, the common tube fluidly coupled to an interior of the sheath and configured to supply fluid thereto and remove fluid therefrom;
a manifold disposed within the housing, the manifold defining an inflow port fluidly coupled to the inflow tube, an outflow port fluidly coupled to the outflow tube, and a common tube port fluidly coupled to the common tube; and
first and second valve assemblies disposed within the housing and operably coupled to the manifold, the first valve assembly configured to selectively control a flow of fluid from the inflow tube to the interior of the sheath, the second valve assembly configured to selectively control a flow of fluid from the interior of the sheath to the outflow tube.

2. The surgical instrument according to claim 1, wherein each of the first and second valve assemblies includes a solenoid valve.

3. The surgical instrument according to claim 2, further comprising a selector assembly including first and second switches configured to selectively operate the solenoid valves of the first and second valve assemblies, respectively, preferably further comprising a toggle switch disposed on the housing and configured to selectively actuate the first and seconds switches.

4. The surgical instrument according to claim 1, 2 or 3, wherein the elongated shaft defines a C-shaped configuration and includes a side-facing opening defined therein along a portion of a length thereof, preferably wherein at least one drive component associated with the end effector assembly extends through the elongated shaft.

5. The surgical instrument according to any preceding claim, wherein the elongated shaft defines a flared distal portion.

6. The surgical instrument according to any preceding claim, wherein the sheath includes an expandable distal portion, preferably further comprising an expandable structure disposed within the expandable distal portion of the sheath and configured to selectively expand the expandable distal portion of the sheath.

7. The surgical instrument according to any preceding claim, further comprising at least one flow director disposed within the sheath adjacent the end effector assembly, the at least one flow director configured to direct a flow of fluid through the sheath around the end effector assembly.

8. A robotic surgical system, comprising:
a robotic arm including a plurality of tube guides disposed thereon; and
a robotic surgical instrument configured to releasably engage the robotic arm, the robotic surgical instrument including:
a housing;
an elongated shaft extending distally from the housing;
an end effector assembly supported at a distal end portion of the elongated shaft;
a sheath disposed about the elongated shaft and movable relative to the elongated shaft and the end effector assembly between a retracted position, wherein the sheath is positioned proximally of the end effector assembly, and an extended position, wherein the sheath is disposed about the end effector assembly;
an inflow tube routed through at least one of the plurality of tube guides and extending into the housing;
an outflow tube routed through at least one of the plurality of tube guides and extending into the housing; and
first and second valve assemblies disposed within the housing and operably coupled to the inflow and outflow tubes, respectively, the first valve assembly configured to selectively control a flow of fluid from the inflow tube to the interior of the sheath, the second valve assembly configured to selectively control a flow of fluid from the interior of the sheath to the outflow tube, preferably wherein the robotic surgical instrument further includes:
a common tube extending into the housing, the common tube fluidly coupled to an interior of the sheath and configured to supply fluid thereto and remove fluid therefrom; and
a manifold disposed within the housing, the manifold defining an inflow port fluidly coupled to the inflow tube, and outflow port fluidly coupled to the outflow tube, and a common tube port fluidly coupled to the common tube.

9. The surgical system according to claim 8, wherein each of the first and second valve assemblies includes a solenoid valve, preferably further comprising first and second switches disposed within the housing and configured to selectively operate the solenoid valves of the first and second valve assemblies, respectively.

10. The surgical system according to claim 8 or 9, wherein the housing includes a main housing body and a fluid control box, the first and second valve assemblies disposed within the fluid control box.

11. The surgical system according to claim 8, 9 or 10 wherein the elongated shaft defines a C-shaped configuration and includes a side-facing opening defined therein along a portion of a length thereof preferably wherein at least one drive component associated with the end effector assembly extends through the elongated shaft.

12. The surgical system according to any one of claims 8 to 11, wherein the elongated shaft defines a flared distal portion.

13. The surgical system according to any one of claims 8 to 12, wherein the sheath includes an expandable distal portion and wherein an expandable structure is disposed within the expandable distal portion, the expandable structure configured to selectively expand the expandable distal portion of the sheath.

14. The surgical system according to any one of claims 8 to 13 , further comprising at least one flow director disposed within the sheath adjacent the end effector assembly, the at least one flow director configured to direct a flow of fluid through the sheath around the end effector assembly.
